Europäisches Patentamt

European Patent Office

Office européen des brevets

(19)

(11) EP 0 494 950 B1

(12) EUROPEAN PATENT SPECIFICATION

(45) Date of publication and mention
of the grant of the patent:
08.05.1996 Bulletin 1996/19

(51) Int Cl.⁶: **C12P 13/00**, C12P 1/02,
C12N 1/19
// C12R1:645

(21) Application number: 90915541.8

(22) Date of filing: 08.10.1990

(86) International application number:
PCT/SE90/00646

(87) International publication number:
WO 91/05869 (02.05.1991 Gazette 1991/10)

(54) **FUNGAL CELL WALL MATERIAL WITH FLOCCULANT PROPERTIES, METHOD FOR ITS PRODUCTION**

PILZZELLWANDMATERIAL MIT FLOCKUNGSEIGENSCHAFTEN UND VERFAHREN ZU SEINER HERSTELLUNG

MATIERE DE MEMBRANE DE CELLULE FONGIQUE PRESENTANT DES CARACTERISTIQUES FLOCULANTES ET PROCEDE POUR SA FABRICATION

(84) Designated Contracting States:
AT BE CH DE DK ES FR GB IT LI NL SE

(30) Priority: 13.10.1989 SE 8903368

(43) Date of publication of application:
22.07.1992 Bulletin 1992/30

(73) Proprietors:
• EDEBO, Lars
S-412 70 Göteborg (SE)
• LI, Xiangpeng
S-421 Va Frölunda (SE)

(72) Inventors:
• EDEBO, Lars
S-412 70 Göteborg (SE)
• LI, Xiangpeng
S-421 Va Frölunda (SE)

(74) Representative: Andersson, Rolf
Berol Nobel AB
Patentavdelningen
S-444 85 Stenungsund (SE)

(56) References cited:
DE-A- 2 923 802

• Agr. Biol. Chem., Vol. 40, No. 3, 1976 JUNJI NAKAMURA et al.: "Purification and Chemical Analysis of Microbial Cell Flocculant Produced by Aspergillus sojae AJ7002", see page 619 - page 624 see p 621, right column, line 1-5 from the bottom - p622.
• CHEMICAL ABSTRACTS, Volume 66, No. 9, 27 February 1967, (Columbus, Ohio, US), P.J. MILL: "Phosphomannans and other components of flocculent and non-flocculent walls of Saccharomycescerevisiae", see page 3376, abstract 35614t, & J. Gen. Microbiol. 1966, 44(3), 329-341
• CHEMICAL ABSTRACTS, Volume 91, No. 21, 19 November 1979, (Columbus, Ohio, US), COLE, GARRY T et al.: "Surface ultrastructure and chemical composition of the cell walls of conidial fungi", see page 329, Abstract 171295h, & Exp. Mycol. 1979, 300(2), 132- 156.
• Process Biochemistry, Vol. 19, 1984 W.J. Mc GAHREN et al.: "Chitosan by fermentation", see page 88 - page 90.
• CHEMICAL ABSTRACTS, Volume 73, No. 21, 23 November 1970, (Columbus, Ohio, US), WAI, NGANSHOU: "Comparison of hexosamine contents in cell walls of Aspergillus and Mucor", see page 100, Abstract 106501m, & Bull., Inst. Chem., Acad. 1970, 17(), 1- 8.
• J. Gen. Microbiol. 44, 1966, 329 - 341
• Römpp Chemie Lexikon, 9th edition, Georg Thieme Verlag, 5124 - 5125

EP 0 494 950 B1

- **Analytical Biochemistry, D.J. Holme & H. Peck, Longman, 1983, 116 - 117**

- **Advanced Cell Biology, L.M. Schwarz & M.M. Azar (eds), Van Nostrand Reinhold, 1981, 373**

## Description

This invention relates to a fungal cell wall material having a positive Zeta-potential at pH 7, a method for the production of the fungal cell wall material as well as its use.

Chitosan is used as a flocculant particularly in waste water treatment as it contains amino groups. Since most biological molecules and cells are negatively charged, waste water material containing organic materials like proteins, nucleic acids, bacteria, fatty acids, cellulose fibres, has been flocculated with positively charged chitosan in order to clarify the waste water.

Generally chitosan has been produced from marine waste residues, e.g. shells of crabs, shrimps, prawn and krills. Firstly, the chitin (polyacetylglucosamine) has been extracted from the shells by acid, then hydrolyzed by concentrated alkaline solution at high temperature to produce chitosan, an alkali insoluble and acid soluble polymer. Chitosan has usually been extracted and solubilized in acid solution. After filtration the liquid was neutralized by alkali such that the chitosan precipitated and was recovered.

The mycelium of zygomycetes and some ascomycetes also contains chitin or chitosan. Chitosan-glucan-complexes have been extracted from the mycelium of Mucor rouxii and Aspergillus niger by 40% sodium hydroxide at 128°C for 4 h. The chitosan-glucan-complex was an acetic acid soluble white powder with amino-groups detected by IR spectrum and with positive charges shown by colloidal titration (Muzzarelli, Ricardo, German Offenlegungsschrift 2 923 802, 1979). Furthermore, fungal chitosan has been reported to have been produced from Absidia coerulea. The mycelium of Absidia coerulea was de-proteinated by 2% sodium hydroxide boiling for 1 h. Then the chitosan was extracted and solubilized from the de-proteinated mycelium by 2% acetic acid, but the acetic acid insoluble material was discarded (W.I. McGahren, etc., Process Biochemistry, 19, 88-90, 1984). Finally the publication J. Gen. Microbiol. 44, 1966, 329-341 and Chemical Abstracts 66, 9, 3367 abstract No 35614 disclose a hexosamine product obtained from a culture broth, and produced by a number of steps involving dissolution of intermediates in acidic media.

According to the present invention it has been found that fungal cell material which is water insoluble both in acidic and alkaline milieu and has a positive Zeta-potential at a pH value of 7, depending on the presence of hexosamines in the cell wall material has excellent flocculant and immobilizing properties. The Zeta-potentials were determined on cell wall materials having a particle size of less than 20 μm in a Malvern Zetasizer. In case the cell wall material of the invention is not in a state of small particles the material has to be disintegrated before the Zeta-potential can be determined. It has still the spongy fungus structure and contains large quantities of glucosamine units. The object of the invention is defined in the claims.

The flocculation ability of the cell wall material, e.g. for proteins such as bovine serum albumin and for nucleic acids such as ribonucleic acid, is much higher than the flocculating ability of chitosan. The cell wall material may easily be regenerated by adding acetic acid to the floccules of the cell wall and the protein. When the pH of the flocculated liquid is decreased by acetic acid, the floccules disappear and the protein dissolves in water. The cell wall material may be recovered by filtration or centrifugation.

The fungal cell wall material according to the invention may be used as a selective flocculant, carrier, and/or ion exchanger in processes to recover or remove negatively charged products like chemical compounds, polymers, e.g. proteins as enzymes and hormones as well as nucleic acids from water based fluids. Furthermore, waste waters such as waste water from slaughterhouses, yeast and margarine factories, fish canning industry, and pulp and paper industry may advantageously be clarified by using the cell wall material as a flocculant.

The fungal cell wall material is also an excellent biological carrier for immobilization of microbial cells and enzymes. The binding forces are often so firm that the immobilized cells and enzymes are stable in high ionic strength, acidic and alkaline water solution as well as in aqueous solutions subjected to vigorous stirring. The cells may be chosen from the group consisting cf bacteria, yeasts, fungal mycelia, plant cells and animal cells, and the enzymes from the group consisting of amylase, catalase, cellulase, deoxyribonuclease, beta-galactosidase, beta-glucanase, glucose oxidase, glucose isomerase, lipase, lysozyme, papain, penicillin acylase, pepsin, protease, ribonuclease, trypsin, urease, and any enzymes from the sources of animals, plants, and microorganisms.

Cells and enzymes having iso-electric points at acidic pH-values can be immobilized on the cell wall material at pH-values around and below 7. In some cases the floccules formed are so stable that it is not necessary to use cross-linking agents. In some cases, e.g. when the cells and enzymes have iso-electric points at neutral or alkaline pH-values, floccules may not be formed or the floccules formed may be insufficiently stable in high ionic strength solution or during vigorous stirring. In these cases bifunctional reagents, such as dialdehydes and diisocyanates may be used for cross-linking in a conventional manner. Independent on whether the immobilization is achieved with or without cross-linking agents the enzymes immobilized in accordance with the present invention showed extraordinary enzyme activity and loading.

The fungal cell wall material according to the invention is prepared from a fungus containing chitin or chitosan by a process wherein the fungus is subjected to extractions in order to remove lipids, proteins, nucleic acids and chitosan. The fungus is normally also physically disintegrated in order to facilitate the extraction. According to a suitable process, the mycelium is

a) physically disintegrated and/or delipidated by treatment with an organic solvent;
b) deproteinated and depleted from nucleic acid by treatment with alkali or enzyme and, if desired, treated with an acid to remove chitosan.

In the process of the invention any fungi whose cell walls contain chitin or chitosan may be used. Examples of suitable fungi are the phylum Zygomycota with the genera Absidia, Mucor and Rhizopus, as well as the phylum Dikarycmycota with the genera Aspergillus, Fusarium and Penicillum. These fungi may be cultured in a liquid medium containing for example a nitrogen source, a carbon source and vitamins. Examples of other nitrogen sources are inorganic sources, such as $(NH_4)_2SO_4$, $NH_4Cl$, $NH_4NO_3$ or organic sources, such as urea, peptone, soybean protein and corn steep liquor. Yeast extract may be added as a vitamin supply. Suitable carbon source is carbohydrates such as glucose, lactose, maltose, sucrose, starch and molasses. The culture conditions are not critical but conventional methods could be applied.

The physical disintegration step can be performed by any conventional method such as sonic treatment, mill grinding, sand vibration and high speed shearing but preferably the disintegration is carried out by freeze-pressing. The disintegrated cell walls have preferably a particle size lower than 20 μm. After the disintegration the intracellular protoplasmic material may be washed out with water.

The disintegrated cell walls are preferably subjected to extraction with an organic solvent such as methanol, ethanol, hexane, chloroform, acetone, and benzene to remove lipids. Among the solvents ethanol is the preferred one.

The de-lipidated cell walls are then treated with alkali or enzymes to remove protein and nucleic acid. Suitable alkali solutions are water solutions of KOH, NaOH, $Ca(OH)_2$, and $NH_4OH$. The enzyme proteinase could be used to remove protein, and the enzyme nuclease to remove nucleic acid. The concentration of the alkali depends on the fungus phylum used. Preferably it is 0.2 N-2.0 N of NaOH for zygomycetes and about 8 N for ascomycetes. When the cell walls are not subjected to physical disintegration or only a mild disintegration resulting in an average particle size larger than 20 μm the treatment with alkali should be performed by using a relatively strong alkali solution, such as 1.0 N of NaOH or higher for zygomycetes.

The removal of chitosan from delipidated, de-proteinated and nucleic acid-depleted cell walls is an optional but preferred step in the process of manufacture of the cell wall material. By acid treatment, free chitosan is dissolved and removed from the cell walls. Acetic acid is the preferred acid but other acids, such as dilute hydrochloric and formic acid, may also be used. Removed chitosan may be precipitated by adjusting the pH to 9.0.

The cell wall material produced by the process of the invention contains hexosamines and exhibits a positive Zeta-potential at acid and neutral pH values and preferably the Zeta-potential is above 20 mV at pH 6.

At pH 7 the physically disintegrated cell walls as well as the de-lipidated cell walls are negatively charged in water. However, after alkali extraction as well as after succeeding acetic acid extraction, the disintegrated cell wall material shows positive Zeta-potential (positive charge). Normally, the disintegrated cell wall of the invention has an average particle size of less than 20 μm.

The cell wall material of the invention had an excellent ability to flocculate bovine serum albumin (BSA) at a pH between 4 and 7 and yeast RNA at a pH between 3.5 and 6.2. Waste waters from slaughterhouses and yeast, margarine and fish canning factories can easily be clarified by the cell wall material at pH 6.

The present invention is further illustrated by the following examples.

Example 1

Five different zygomycetes strains and two different ascomycetes strains according to Table 1 below were cultured in a medium containing glucose, yeast extract and mineral salts. After growth, the mycelium was filtrated out on a nylon net and disintegrated in an X-press. The disintegrated mycelium was washed with water and then extracted with hot ethanol, twice with hot 1N NaOH for zygomycetes and 8N NaOH for ascomycetes, twice with hot water and finally with 0.2 N acetic acid, each extraction step being followed by filtration. The hexosamine content as detected by a modified Elson-Morgan method (George C. Chen etc. Appl. Envir. Microbiol. 46, 13-16, 1983) and Zeta-potentials (Malvern Zetasizer II, Malvern Instruments Ltd, Malvern, Worcestershire WR14 1AQ, England) of the cell wall materials obtained are shown in Table 2 below as well as the yields of the cell wall material and chitosan. Most cell wall material had a particle size of 5-10 μm.

Table 1

| A | Absidia coerulea IMI 38500 |
|---|---|

Continuation of the Table on the next page

Table 1   (continued)

| | |
|---|---|
| B | Mucor sp. (Chinese isolate) |
| C | Mucor rouxii ATCC 24905 |
| D | Rhizopus oligosporus MUCL 18813 |
| E | Rhizopus oryzae CBS 112.07 |
| F | Aspergillus niger CCUG sp. |
| G | Fusarium poae CCUG 22425 |

ATCC =   American Type Culture Collection, 12301 Parklawn Drive, Rockville, Maryland 20852, USA.
CBS =    Centraalbureau voor Schimmelcultures, Julianalaan 67a, 2628 BC Delft, Netherlands.
CCUG = Culture Collection, University of Göteborg, Guldhedsgatan 10, S-413 46 Göteborg, Sweden.
IMI =     Commonwealth Mycological Institute, Kew, Surrey, England.
MUCL = Mycotheque de l'Université Catholique de Louvain, Place Croix du Sud 3 Bte 8, 1348 Lourain-la-Neuve, Belgium.

Table 2

| Strain (see Table 1) | A | B | C | D | E | F | G |
|---|---|---|---|---|---|---|---|
| Yield of cell wall material, % by weight | 10.8 | 9.59 | 8.11 | 8.82 | 7.08 | 9.03 | 15.9 |
| Yield of chitosan, % by weight | 10.2 | 5.61 | 2.97 | 7.77 | 11.04 | 1.97 | 0.32 |
| Hexosamine in cell wall material, % by weight | 60.0 | 66.5 | 52.6 | 50.6 | 59.4 | 13.8 | 38.1 |
| Zeta-potential, mV pH 7.0 pH 6.0 pH 4.0 | 18.4 31.3 78.0 | 9.2 30.0 42.8 | 14.5 24.9 64.4 | 6.8 25.8 31.2 | 7.6 28.0 45.0 | 13.6 25.1 49.0 | 18.7 40.0 59.2 |
| Iso-electric point | 7.8 | 7.3 | 7.5 | 7.6 | 7.4 | 8.0 | 8.5 |

As a comparison, crude cell wall material was prepared from Strain B and Strain E by disintegration and thoroughly washing the disintegrated cell walls in water. Part of the disintegrated cell wall material was extracted with hot ethanol to obtain delipidation. The Zeta-potentials of both the delipidated cell wall material and crude cell wall material were measured. The following results were obtained.

Table 3

| | | Zeta-potential, mV | |
|---|---|---|---|
| | | Strain B | Strain E |
| Crude cell wall | pH 4 | - 11.7 | - 12.7 |
| Delipidated cell wall | pH 4 | - 16.1 | - 14.1 |
| Crude cell wall | pH 6 | - 60.2 | - 35.0 |
| Delipidated cell wall | pH 6 | - 37.7 | - 27.3 |

From the above it is evident that the crude cell wall materials at a pH-value above 4 exhibit negative Zeta-potentials, while cell wall materials produced in accordance with the invention are positively charged.

Another comparison test was also performed in which the mycelium was treated in accordance with W. I. McGahren, etc., Process Biochemistry, 19, 88-90, 1984. After disintegration in a Waring blendor the Zeta-potential was measured and found negative at pH 7.0.

Example 2

3.6 mg dry weight of cell wall material from Example 1 and one ml bovine serum albumin BSA (6 mg/ml) were pipetted into a 10 ml test tube. Water was added in such an amount that after pH adjustment to a pH value of 6.0, the suspension volume became 6 ml. The final BSA concentration was 1 mg/ml and the cell wall material was 600 ppm.

When the positively charged cell wall materials from the tests of Example 1 were mixed with BSA, flocculation and aggregation appeared immediately. No flocculation or aggregation took place when the negatively charged crude cell wall material and de-lipidated cell material from the comparison in Example 1 were used instead of the positively charged cell wall material. Comparison tests with chitosan were also performed. The flocculation ability was determined with UV absorbancy at 278 nm and was calculated as follows:

$$\text{Ability of flocculation } (\%) = 100 - \frac{\text{O.D. (supernatant)}}{\text{O.D. (BSA control)}} \times 100$$

The following results were obtained.

Table 4

| Fungus | BSA flocculation ability % |
|--------|----------------------------|
| A[1] | 97 |
| B | 95 |
| C | 99 |
| D | 96 |
| E | 95 |
| F | 91 |
| G | 90 |
| H[2] | 77 |
| Chitosan[3] | 58 |
| Chitosan[4] | 18 |

[1] Letters A-G refer to Table 1.

[2] Penicillium frequentans CCUG sp.

[3] Maximum ability at a concentration of 50-100 ppm.

[4] Maximum ability at a concentration of about 300 ppm.

From the tests it is evident that the cell wall materials of the invention have excellent BSA flocculation abilities and are superior to chitosan in this respect.

Example 3

Rhizopus oryzae CBS 112.07 was cultured and harvested in the same manner as in Example 1. The mycelium was washed and extracted directly with hot ethanol for 3 h. After drying, de-lipidated mycelium was obtained and 15 g thereof was treated by NaOH and acetic acid largely in a similar manner as in Example 1, but the concentration of the NaOH was 0.2 N. After washing with water, the alkali-treated mycelium was suspended in 0.2 N acetic acid and disintegrated under high shear force (Ultra turrax; Janke & Kunkel, Staufen, West Germany) for 1 min which yielded 300 ml of 4.12 mg/ml of bioflocculant. After centrifugation and pH adjustment to 9.0 of the aqueous liquid phase, chitosan was obtained it a yield of 7.65% by weight. The yield of cell wall material according to the invention was 6,77% by weight. Flocculation of BSA was performed as in Example 2 with different concentrations of cell wall bioflocculant from Rhizopus oryzae CBS 112.07. The results obtained are shown in Table 5.

Table 5

| Cell wall material, ppm | 100 | 200 | 400 | 600 | 800 |
|--------------------------|-----|-----|-----|-----|-----|
| BSA flocculation | 38 | 65 | 95 | 95 | 94 |

Example 4

Ribonucleic acid (RNA) solution (purity 78%, 0.5 mg/ml in 0.05 % NH$_4$OH and 10 % NaCl) derived from yeast in an amount of 1 ml was added together with 2.75 ml cell wall material (1.6 mg/ml) prepared from Rhizopus oryzae CBS 112.07 as in Example 1 and 0.1 ml 2 N acetic acid into test tubes. The pH-values of the mixtures were adjusted by adding 0.1 N NaOH. The flocculation ability was determined. RNA was detected by UV absorbancy at 260 nm after hydrolysis with 0.5 N perchloric acid at 70°C (S. Ohla et al, Applied Microbiology 22, p 415-421, 1971). The following results were obtained.

6

Table 8

| pH | 3.5 | 4.0 | 4.5 | 5.0 | 5.5 | 6.2 |
|---|---|---|---|---|---|---|
| RNA flocculation, % | 89 | 94 | 95 | 97 | 97 | 97 |

From the results it is evident that the cell wall material had an excellent flocculation ability on RNA. As a comparison, chitosan did not flocculate RNA at wide ranges of concentrations at the above pH values.

Example 5

Flocculation of BSA with cell wall bioflocculant was performed as in Example 2 at pH 6.0. The cell wall bioflocculants of the Mucor sp. (Chinese isolate) and Rhizopus oryzae CBS 112.07 were regenerated three times as follows. The BSA-bioflocculant floccules were centrifuged and resuspended in 5 ml 0.2 N HAc, vigorously agitated for 20 min, centrifuged at 4000 rpm for 10 min, and resuspended in 5 ml 0.2 N HAc, and agitated again for 1 min. After centrifugation the sediment was washed twice with 10 ml water and finally centrifuged at 4000 rpm for 15 min. The regenerated cell wall bioflocculant was re-used to flocculate BSA.

Table 9

| The regeneration of cell wall bioflocculant. Flocculation condition: BSA 1 mg/ml, cell wall bioflocculant 1.2 mg/ml; pH 6.0. | | |
|---|---|---|
| Cell wall bioflocculant | Time of utilization | BSA flocculation % |
| Mucor sp. (Chinese isolate) | | |
| | 1 | 95.1 |
| | 2 | 98.6 |
| | 3 | 99.1 |
| | 4 | 99.7 |
| Rhizopus oryzae CBS 112.07 | | |
| | 1 | 95.2 |
| | 2 | 97.2 |
| | 3 | 98.5 |
| | 4 | 98.6 |

From the results it is evident that the flocculation ability was not influenced adversely by the regeneration.

Exampel 6

Waste water from a yeast factory, a margarine factory, a slaughterhouse and a fish canning factory were clarified by cell wall bioflocculants. The cell wall bioflocculant suspensions of Rhizopus oryzae CBS 112.07 or the Mucor sp. (Chinese isolate) were prepared as in Example 1. The flocculant suspensions were added to 50 ml water samples during 20 see with high stirring speed to a final volume of 60 ml (100 ml with the water from the fish canning factory). The pH was adjusted to 6.0-6.2 with NaOH or HCl and the samples were stirred slowly for 2 min. The samples were left for settling during 75 min (180 min with the fish canning factory water), whereafter the top 25 ml were sucked off with a pipette for turbidity measurements. The results of the waste water clarification, shown as residual turbidity, are given in the table below.

Table 10

| Flocculant Turbidity Residual turbidity (FTU) | | | | | | | |
|---|---|---|---|---|---|---|---|
| Waste water from | (FTU) Control | Mucor sp, mg/l | | | Rhizopus oryzae mg/l | | |
| | | 50 | 100 | 400 | 50 | 100 | 400 |
| Yeast factory | 23 | 21 | 18 | 7 | 20 | 16 | 8 |
| Margarine factory | 51 | 27 | 18 | 10 | 18 | 6 | 10 |

Continuation of the Table on the next page

Table 10 (continued)

| Flocculant Turbidity Residual turbidity (FTU) | | | | | | | |
|---|---|---|---|---|---|---|---|
| Waste water from | (FTU) Control | Mucor sp, mg/l | | | Rhizopus oryzae mg/l | | |
| | | 50 | 100 | 400 | 50 | 100 | 400 |
| Slaughterhouse | 42 | 17 | 12 | 9 | 11 | 10 | 11 |
| Fish canning | -* | 90 | 27 | 17 | 29 | 22 | 23 |
| * not measured | | | | | | | |

Example 7

0.25 ml penicillin V acylase (78 U/ml, 5.0 U/mg protein), 1.55 ml cell wall material at 5.0 mg/ml (Rhizopus oryzae, CBS 112.07 produced in accordance with Example 1), and 1 ml of 50 mM potassium phosphate buffer, pH 7.5 (KPB), were pipetted into a 10 ml test tube, whereupon different amounts of 2.5 % glutaraldehyde and finally distilled water were added to make a total volume of 5.0 ml. The final glutaraldehyde concentration varied from 0.04 % to 0,16 %. All mixtures were shaken overnight at 4°C. The immobilized penicillin V acylase was recovered by centrifugation. The sediment was washed twice with 10 ml distilled water, once with 2 ml 3 M NaCl, and twice with 10 ml distilled water in succession and finally suspended in distilled water to 5.0 ml, mixed thoroughly and the activity of immobilized penicillin V acylase was determined as follows:

Enzyme, ca 5 U of 1 ml immobilized enzyme suspension, distilled water 20 ml, and 2.0 ml 50 mM KPB, pH 7.5 were mixed and stirred vigorously at 28°C, and then 20 ml 4 % potassium penicillin V (distilled water solution) was added. The pH was readjusted to 7.5 with titration of 10 mM NaOH after 10 min. One unit of penicillin V acylase is defined as the production of 1 μM 6-aminopenicillanic acid in one minute. The influence of the concentration of glutaraldehyde onto the activity of immobilized penicillin V acylase is shown in table 11 and the ratio between enzyme and cell wall in table 12.

Table 11

| Glutaraldehyde % | 0.04 | 0.08 | 0.12 | 0.16 |
|---|---|---|---|---|
| Immobilized activity U/g dry cell wall | 1330 | 1650 | 1640 | 1600 |
| Immobilized penicillin V acylase % | 52.8 | 65.4 | 65.1 | 63.6 |

Table 12

| The procedure was the same as the one used for the tests shown in table 11 but different volumes of cell wall suspension at 10 mg/ml, and glutaraldehyde in an amount giving a concentration of 0.1 % by weight were used. | | | | | | | | |
|---|---|---|---|---|---|---|---|---|
| Acylase/cell wall (w/w) | 0.15 | 0.20 | 0.25 | 0.30 | 0.40 | 0.50 | 0.75 | 1.00 |
| Immobilized activity (U/g dry cell wall) | 523 | 717 | 919 | 1149 | 1435 | 1723 | 2321 | 2555 |
| Immobilized penicillin V acylase (%) | 69.2 | 71.3 | 73.1 | 76.2 | 71.3 | 68.5 | 61.5 | 50.8 |

Example 8

1.0 g of pressed Baker's yeast, Saccharomyces cerevisiae, with 25% dry weight was suspended in 100 ml 0.01 M phosphate buffer pH 6.0. Two ml of the yeast suspension and cell wall material (produced in accordance with Example 1, Rhizopus oryzae CBS 112.07 5.0 mg/ml) were pipetted into a 10 ml test tube in such amounts that the ratios stated in Table 13 were obtained. The ratio (w/w) of dry pressed yeast to cell wall material was maintained from 0.5 to 3.0. Phosphate buffer 0.01 M, pH 6.0 was added to 5.0 ml and the suspensions were mixed thoroughly. Flocculation appeared immediately. Microscopically the floccules consisted of yeast cells intermingled with cell wall material. All the 5.0 ml suspension was filtered through a filter paper and the turbidity of the filtrate was determined at 500 nm. The immobilization of the yeast cells was calculated as follows:

$$\text{Immobilization (\%)} = 100 \, (1 - OD_{filtrate}/OD_{control})$$

The following results were obtained:

Table 13

| Pressed yeast/cell wall (w/w) | 0.5 | 1.0 | 1.5 | 2.0 | 2.5 | 3.0 |
|---|---|---|---|---|---|---|
| Immobilization of yeast cells % | 98.8 | 99.5 | 99.7 | 98.7 | 89.5 | 86.0 |

The yeast cells immobilized with the cell wall material were stable during vigorous stirring, high ionic strength and acidic or alkaline water solutions, e.g. during vigorous stirring at 500 rpm, in distilled water, 2% NaCl, 0.1 M phosphate buffer pH 8.0, 1% $NH_4OH$, 0.1 N HAc, 0.1 M $H_3PO_4$, 0.1 M HCl at room temperature. The immobilized yeast cells could be treated with pressed filtration, vacuum filtration or centrifugation. In comparison the flocculation of yeast cells with chitosan was not stable in 2% NaCl solution nor in distilled water.

Example 9

Pseudomonas putida EP 12690 from CCUG was cultured in meat extract medium, harvested by centrifugation, washed with water twice, and suspended in water at a dry weight of 10.8 mg/ml. 0.5 ml of the bacterial suspension and cell wall material (produced in accordance with Example 1 Rhizopus oryzae CBS 112.07) were pipetted into a 10 ml test tube in such amounts that the weight ratios stated in Table 14 were obtained, and water was added to 5.0 ml and the immobilization procedure and determination of immobilization performed as for immobilized yeast cells in Example 1.

The following results were obtained:

Table 14

| Bacteria/cell wall (ω/w) | 0.55 | 0.68 | 0.90 | 1.35 | 2.70 |
|---|---|---|---|---|---|
| Immobilization of bacterial cells % | 92 | 95 | 96 | 97 | 61 |

The cell wall preparation and Pseudomonas putida are both given as dry weight. The floccules of Pseudomonas putida and cell walls were as stable as those of baker's yeast during high speed stirring at 500 rpm, filtration and centrifugation procedures, in distilled water, 2% NaCl, 0.1 M phosphate buffer pH 8.0, 1% $NH_4OH$, 0.1 N HAc, 0.1 M $H_3PO_4$ and 0.1 N HCl.

The loading capacity to immobilize microbial cells to cell wall material was high, 1.0-2.0 g yeast cells or 0.9-1.35 g bacterial cells per g cell wall mateial (dry wt.). It is evident that cell wall material of this invention is a good matrix for microbial cell immobilization, because of high loading and stability, even in strong acidic and alkaline water solutions.

Exemple 10

Jack bean urease 75 U/mg (Sigma Chemical Co) was dissolved in 0.02 M phosphate buffer pH 7.0 at 1 mg/ml. Four ml of the urease solution and 4 mg/ml cell wall material (produced in accordance with Example 1, Absidia coerulea IMI 38500) were pipetted into a 10 ml test tube in such amounts that the weight ratio stated in Table 15 were obtained, slowly stirred for 5 min and then left overnight at 4°C. The cell walls with bound urease were recovered by centrifugation. The urease activity of the supernatant was determined and considered as not immobilized urease. The precipitate was washed with 5 ml distilled water, 5 ml 3 N NaCl, 5.0 ml distilled water x 2 and 5 ml 0.02 M phosphate buffer pH 7.0 in succession and finally suspended in 4.0 ml 0.02 M phosphate buffer pH 7.0, mixed thoroughly and used as immobilized urease preparation. The activities of the supernatants as free (not immobilized) urease and immobilized urease were detected as stated in Worthington Enzyme Manual, p 144, Worthington Biochemical Co., Freehold, New Jersey, USA, 1972. One unit of urease activity was defined as liberating one micromole of ammonia per minute at pH 7.0 and 25°C. The free urease in the supernatant amounted to 0.9-3.4 %. The following results were obtained.

Table 15

| urease/cell wall (w/w) | 2:3 | 1:2 | 1:3 |
|---|---|---|---|
| Immobilized urease % | 75.6 | 75.6 | 75.6 |
| Immobilized activity U/g dry cell wall | 37800 | 28350 | 18900 |

Immobilized urease is given as % of the initial urease activity before immobilization.

The stability of cell wall immobilized urease at 4°C and room temperature is shown in Table 16.

Table 16

| Day<br>Activity % | 0 | 11 | 27 | 42 |
|---|---|---|---|---|
| 4°C | 100 | 89 | 78 | 72 |
| Room temp. | 100 | 89 | 71 | 34 |

The enzyme activity of the immobilized urease was stable under vigorous stirring conditions. The urease loading 37800 U/g (dry cell wall) was much higher than that of hydrophilic, synthetic porous polymer beads 416 U/g (dry support) (Mauz, Otto, etc., Ger. Offen. DE 3 714 276, 1988) and that of a synthetic cation exchange resin 340 U/g (dry resin) (H.J. Moynihan, etc., Biotechnology and Bioengineering 34:951-963, 1989) . The half-lives of the enzyme activity of urease immobilized to cell wall were more than 27 days both at 4°C and at room temperature.

Example 11

Glucose oxidase 59.4 U/mg was dissolved in distilled water at 2 mg/ml. Four ml of the glucose oxidase solution, 1.6 ml cell wall material (5.0 mg/ml Rhizopus oryzae CBS 112.07 in accordance with Example 1), 2.4 ml distilled water and 0.32 ml of 5 % glutaraldehyde were pipetted into a 10 ml test tube, mixed and then left overnight at 4°C. The glucose oxidase was fixed to the cell wall material using the bifunctional compound glutaraldehyde as cross-linking reagent. The cell wall immobilized glucose oxidase was recovered by centrifugation. The precipitate was washed with 10 ml distilled water x 2, 10 ml 0.25 M KCl, 10 ml distilled water x 2 in succession and finally suspended in 200 ml distilled water. This 200 ml suspension was homogenized under high shear force for 1 minute and the immobilized glucose oxidase was determined (Worthington Enzyme Manual, p 19, Worthington Biochemical Co., Freehold, New Jersey, USA, 1972). One unit of glucose oxidase activity was defined as the quantity liberating one micromole of $H_2O_2$ per minute at 25°C.

The loading of immobilized glucose oxidase was 26800 U/g (dry cell wall) which is much superior to that of synthetic polyacrylamine beads 100 U/g (dry support) (B. Szajáni, etc., Applied Biochemistry and Biotechnology 14:37-47, 1987). The immobilized glucose oxidase showed 45.1 % of the activity compared to the initial solution of glucose oxidase before immobilization.

Example 12

100 ml cell wall material (6.0 mg/ml Absidia coerulea IMI 38500 in accordance with Example 1), 20 ml phosphate buffer 0.1 M, pH 7.6 and 5.0 ml glutaraldehyde 25 % were mixed in a 200 ml beaker, slowly stirred for 5 min and then left for 2 h at room temp.

The glutaraldehyde activated cell wall material was recovered by vacuum filtration in a sintered glass funnel and washed with 100 ml distilled water 5 times, 50 ml 0.1 M phosphate buffer, pH 7.6 and finally suspended in 60 ml 0.1 M phosphate buffer, pH 7.6. Crude papain 300 ml with 1.5 U/mg was dissolved in 27 ml distilled water solution containing cysteine 0.05 M, EDTA $5x10^{-3}$M and mercaptoethanol $1x10^{-6}$M. The papain solution and the activated cell wall material were mixed and then left overnight at 4°C. The cell-wall immobilized papain was recovered by centrifugation and washed with 50 ml distilled water x 2, 50 ml 1 x NaCl x 2 and 50 ml distilled water x 2 in succession and suspended in distilled water to make a final weight of 20 g altogether.

The activity of immobilized papain was determined (Worthington Enzyme Manual, p 134, Worthington Biochemical Co., Freehold, New Jersey, USA, 1972). One unit of papain activity was defined as hydrolyzing one micromole of benzoyl arginine ethyl ester per minute at 25°C.

The loading of immobilized papain was 370 U/g (dry cell wall) or 49 % of the activity compared to the initial crude papain before immobilization, which is higher than that of p-benzoquinone activated cellulose, 72.4 U/g (dry support) reported by A. Telefoncu, etc., Biotechnology and Bioengineering 23:1689-1674, 1981.

**Claims**

1. Fungal cell wall material being water insoluble in acidic and alkaline milieu, characterized in that it contains hexosamine and exhibits a positive Zeta-potential at a pH value of 7, when disintegrated to a particle size less than 20 μm, the fungal cell material being obtainable from a fungus containing chitin or chitosan by a) physically disintegration and/or delipidation with an organic solvent, and b) de-proteination and depletion from nucleic acid by alkali treatment and/or by treatment with an enzyme.

2. Fungal cell wall material according to claim 1, wherein the content of hexosamine is at least 5% by weight.

3. Fungal cell wall material according to claim 1 or 2, wherein the cell wall material is derived from a fungus selected from the phylum Zygomycota or the phylum Dikaryomycota.

4. Fungal cell wall material according to claim 3, wherein the material is derived from a mycelium.

5. Fungal cell wall material according to any one of claims 1-4, wherein the material at pH 6 has a Zeta-potential of at least 20 mV when disintegrated to a particle size less than 20 μm.

6. Fungal cell wall material in accordance with any one of claims 1-5, wherein the material has an average particle size of less than 20 μm.

7. A process for the manufacture of a fungal cell wall material according to claim 1, characterized in, that the material is obtained from a fungus containing chitin or chitosan by a) physically disintegration and/or delipidation with an organic solvent, and b) de-proteination and depletion from nucleic acid by alkali treatment and/or by treatment with an enzyme.

8. A process in accordance with claim 7, wherein the fungus in step a) is physically disintegrated to a particle size less than 20 μm.

9. A process in accordance with claim 7 or 8, wherein the deproteinated cell wall material is extracted with acids in order to remove chitosan.

10. A process in accordance with any one of claims 7-9, wherein the fungus is selected from the phylum Zygomycota or the phylum Dikaryomycota.

11. A process in accordance with any one of claims 7-10, wherein the cell wall material is obtained from a mycelium.

12. Use of the cell wall material in accordance with any of claims 1-6 in a process to recover or remove negatively charged products from water based media.

13. Use in accordance with claim 12, wherein a cell or a biologically active compound is immobilized on the cell wall material.

14. Use in accordance with claim 13, wherein the cell belongs to the group consisting of bacteria, yeasts, fungal mycelia and plant and animal cells.

15. Use in accordance with claim 13, wherein the compound is an enzyme belonging to the group consisting of amylase, catalase, cellulase, deoxyribonuclease, beta-galactosidase, beta-glucanase, glucose oxidase, glucose isomerase, lipase, lysozyme, papain, penicillin acylase, pepsin, protease, ribonuclease, trypsin, urease and any enzymes from the sources of animals, plants and microorganisms.

16. Use in accordance with claims 13-15, wherein the immobilization is carried out without the presence of any cross-linking agent.

17. Use in accordance with claims 13-15 wherein the immobilization is carried out in the presence of a cross-linking agent.

**Patentansprüche**

1. Pilzzellwandmaterial, das in saurem und alkalischem Milieu wasserunlöslich ist, dadurch gekennzeichnet, daß es Hexosamin enthält und bei einem pH-Wert von 7 ein positives Zeta-Potential aufweist, wenn es auf eine Teilchengröße von weniger als 20 μm zerteilt ist, wobei das Pilzzellmaterial aus einem Pilz, der Chitin oder Chitosan enthält, erhältlich ist durch

   a) physikalische Zerteilung und/oder Entfernung von Lipiden mit einem organischen Lösungsmittel und

b) Entfernung von Proteinen und Nukleinsäure durch Alkalibehandlung und/oder Behandlung mit einem Enzym.

2. Pilzzellwandmaterial nach Anspruch 1, worin der Gehalt an Hexosamin mindestens 5 Gewichtsprozent beträgt.

3. Pilzzellwandmaterial nach Anspruch 1 oder 2, wobei das Zellwandmaterial aus einem Pilz stammt, der aus der Ordnung Zygomycota oder der Ordnung Dikaryomycota ausgewählt ist.

4. Pilzzellwandmaterial nach Anspruch 3, wobei das Material aus einem Myzel stammt.

5. Pilzzellwandmaterial nach irgendeinem der Ansprüche 1-4, wobei das Material bei pH 6 ein Zeta-Potential von mindestens 20 mV aufweist, wenn es auf eine Teilchengröße von weniger als 20 μm zerteilt ist.

6. Pilzzellwandmaterial nach irgendeinem der Ansprüche 1-5, wobei das Material eine durchschnittliche Teilchengröße von weniger als 20 μm aufweist.

7. Verfahren zur Herstellung eines Pilzzellwandmaterials nach Anspruch 1, dadurch gekennzeichnet, daß das Material aus einem Pilz, der Chitin oder Chitosan enthält, erhalten wird durch

a) physikalische Zerteilung und/oder Entfernung von Lipiden mit einem organischen Lösungsmittel und
b) Entfernung von Proteinen und Nukleinsäure durch Alkalibehandlung und/oder Behandlung mit einem Enzym.

8. Verfahren nach Anspruch 7, worin der Pilz in Schritt a) physikalisch auf eine Teilchengröße von weniger als 20 μm zerteilt wird.

9. Verfahren nach Anspruch 7 oder 8, worin das deproteinierte Zellwandmaterial zur Entfernung von Chitosan mit Säuren extrahiert wird.

10. Verfahren nach irgendeinem der Ansprüche 7-9, worin der Pilz aus der Ordnung Zygomycota oder der Ordnung Dikaryomycota ausgewählt ist.

11. Verfahren nach irgendeinem der Ansprüche 7-10, worin das Zellwandmaterial aus einem Myzel erhalten wird.

12. Verwendung des Zellwandmaterials nach irgendeinem der Ansprüche 1-6 in einem Verfahren zur Gewinnung oder Entfernung von negativ geladenen Produkten aus Medien auf Wasserbasis.

13. Verwendung nach Anspruch 12, worin eine Zelle oder eine biologisch aktive Verbindung auf dem Zellwandmaterial immobilisiert ist.

14. Verwendung nach Anspruch 13, worin die Zelle zu der aus Bakterien, Hefen, Pilzmyzelien und Pflanzen- und Tierzellen bestehenden Gruppe gehört.

15. Verwendung nach Anspruch 13, worin die Verbindung ein Enzym ist, das zu der aus Amylase, Katalase, Cellulase, Desoxyribonuklease, β-Galactosidase, β-Glucanase, Glukoseoxidase, Glukoseisomerase, Lipase, Lysozym, Papain, Penicillinacylase, Pepsin, Protease, Ribonuklease, Trypsin, Urease und irgendwelchen Enzymen, die von Tieren, Pflanzen und Mikroorganismen stammen, bestehenden Gruppe gehört.

16. Verwendung nach den Ansprüchen 13-15, worin die Immobilisierung ohne die Anwesenheit eines Vernetzungsmittels durchgeführt wird.

17. Verwendung nach den Ansprüchen 13-15, worin die Immobilisierung in Anwesenheit eines Vernetzungsmittels durchgeführt wird.

**Revendications**

1. Matière de paroi de cellule fongique insoluble dans l'eau dans des milieux acides et alcalins, caractérisée en ce qu'elle contient de l'hexosamine et présente un potentiel zêta positif à une valeur du pH de 7, lorsqu'elle est désintégrée à une taille particulaire inférieure à 20 µm, la matière de cellule fongique pouvant être obtenue à partir d'un fongus contenant de la chitine ou du chitosane par a) désintégration physique et/ou délipidation avec un solvant organique, et b) déprotéination et appauvrissement en acide nucléique par traitement avec un alcali et/ou par traitement avec une enzyme.

2. Matière de paroi de cellule fongique selon la revendication 1, dans laquelle la teneur en hexosamine est d'au moins 5 % en poids.

3. Matière de paroi de cellule fongique selon la revendication 1 ou 2, dans laquelle la matière de paroi de cellule est dérivée d'un fongus sélectionné parmi le phylum Zygomycota et le phylum Dikaryomycota.

4. Matière de paroi de cellule fongique selon la revendication 3, dans laquelle la matière est dérivée d'un mycélium.

5. Matière de paroi de cellule fongique selon l'une quelconque des revendications 1 à 4, dans laquelle la matière à pH de 6 a un potentiel zêta d'au moins 20 mV lorsqu'elle est désintégrée à une taille particulaire inférieure à 20 µm.

6. Matière de paroi de cellule fongique selon l'une quelconque des revendications 1 à 5, dans laquelle la matière a une taille particulaire moyenne inférieure à 20 µm.

7. Procédé de fabrication d'une matière de paroi de cellule fongique selon la revendication 1, caractérisé en ce que la matière est obtenue à partir d'un fongus contenant de la chitine ou du chitosane par a) désintégration physique et/ou délipidation avec un solvant organique, et b) déprotéination et appauvrissement en acide nucléique par traitement avec un alcali et/ou par traitement avec une enzyme.

8. Procédé selon la revendication 7, dans lequel le fongus du stade a) est physiquement désintégré à une taille particulaire inférieure à 20 µm.

9. Procédé selon la revendication 7 ou 8, dans lequel la matière de paroi de cellule déproteinée est extraite par des acides pour éliminer le chitosane.

10. Procédé selon l'une quelconque des revendications 7 à 9, dans lequel le fongus est sélectionné parmi le phylum Zygomycota ou le phylum Dikaryomycota.

11. Procédé selon l'une quelconque des revendications 7 à 10, dans lequel la matière de paroi de cellule est obtenue à partir d'un mycélium.

12. Utilisation de la matière de paroi de cellule selon l'une quelconque des revendications 1 à 6 dans un procédé permettant de récupérer ou d'éliminer des produits chargés négativement de milieux à base d'eau.

13. Utilisation selon la revendication 12, dans laquelle une cellule ou un composé biologiquement actif est immobilisé (e) sur la matière de la paroi de la cellule.

14. Utilisation selon la revendication 13, dans laquelle la cellule appartient au groupe comprenant les bactéries, les levures, les mycéliums fongiques et les cellules végétales et animales.

15. Utilisation selon la revendication 13, dans laquelle le composé est une enzyme appartenant au groupe comprenant l'amylase, la catalase, la cellulase, la désoxyribonucléase, la bêta-galactosidase, la bêtaglucanase, la glucose oxydase, la glucose isomérase, la lipase, le lysosyme, la papaïne, la pénicilline acylase, la pepsine, la protéase, la ribonucléase, la trypsine, l'uréase et n'importe quelle enzyme tirée de sources animales et végétales et de microorganismes.

16. Utilisation selon les revendications 13 à 15, dans laquelle l'immobilisation est réalisée en l'absence d'un agent de réticulation.

17. Utilisation selon les revendications 13 à 15, dans laquelle l'immobilisation est réalisée en présence d'un agent de réticulation.